# EUROPEAN PATENT SPECIFICATION

(11) **EP 2 386 650 B1**
(45) Date of publication and mention of the grant of the patent: **03.07.2013**
(21) Application number: 06007373.1
(22) Date of filing: 07.04.2006
(51) Int. Cl.: C12P 13/08, C12N 15/82

(54) **Method for producing L-amino acids using the gap promoter**
Verfahren zur Herstellung von L-Lysin unter Verwendung des gap Promotors
Procédé de production de L-Lysine comprenant l'utilisation du promoteur gap

(43) Date of publication of application: 16.11.2011
(73) Proprietor: Evonik Degussa GmbH, 45128 Essen (DE)
(72) Inventor: Bathe, Brigitte, Dr., 33154 Salzkotten (DE); Claes, Wilfried, Dr., 33615 Bielefeld (DE); Hans, Stephan, 49078 Osnabrück (DE)

(56) References cited:
- WO-A-02/40679
- WO-A-2005/059144
- CREMER J ET AL: "CONTROL OF THE LYSINE BIOSYNTHESIS SEQUENCE IN CORYNEBACTERIUM GLUTAMICUM AS ANALYZED BY OVEREXPRESSION OF THE INDIVIDUAL CORRESPONDING GENES" APPLIED AND ENVIRONMENTAL MICROBIOLOGY, WASHINGTON,DC, US, vol. 57, no. 6, 1 June 1991 (1991-06-01), pages 1746-1752, XP000616281 ISSN: 0099-2240
- DATABASE BIOSIS [Online] BIOSCIENCES INFORMATION SERVICE, PHILADELPHIA, PA, US; August 2005 (2005-08), ZHAO ZHI ET AL: "Expression of feedback-resistant aspartate kinase gene in Corynebacterium crenatum" XP002387410 Database accession no. PREV200510258313 & WEISHENGWU XUEBAO, vol. 45, no. 4, August 2005 (2005-08), pages 530-533, ISSN: 0001-6209

## Description

L-amino acids are used in the food sector, as pharmaceuticals and for animal nutrition. L-lysine is mainly used as additive for the preparation of feed.

The current world production for L-lysine is estimated to be more than 350 thousand tons a year. The production is based on fermentation by Corynebacterium glutamicum. Review articles concerning various aspects of L-amino acids can be found in volume 79 of Advances in Biochemical Engineering Biotechnology (volume editors: R. Faurie and J. Thommel) entitled "Microbial Production of L-Amino Acids".

The microbial L-lysine biosynthesis by Corynebacterium glutamicum is a well regulated process. In addition to increasing the copy number of a gene the enhancement of enzyme activities can also be achieved by raising the expression value of a gene.

Recent patent applications demonstrate the use of various promoters for the overexpression of genes involved in L-amino acid biosyntheses or central metabolism (WO 2005/059144; WO 2005/059143; WO 2005/059093; WO 2006/008100; WO 2006/008101; WO 2006/008102; WO 2006/008103; WO 2006/008097; WO 2006/008098; US2006/0003424; WO 02/40679 ).

Now it was found that the expression of a lysC gene encoding a feed back resistant aspartokinase was enhanced significantly by cloning the promoter of the gap in front of the lysC gene. The gap promoter is disclosed by Patek et al. (see page 313 in: Journal of Biotechnology 104 (2003) 311-323). The sequence consisting of the gap promoter and the ribosome binding site can be obtained under the access number NC_006958 at the National Center for Biotechnology Information (NCBI, Bethesda, MD, USA). Under this access number the applied sequence spans from position 1685331 to position 1685094. This sequence is referred to as gap-RBS.

A polynucleotide comprising the gap promoter and the coding sequence of the lysC gene was cloned into plasmid pKl8mobsacB (see WO03/014330 for experimental details). As ribosome binding site (RBS) the RBS of the gap gene or of the lysC gene may be used. The exchange of the natural lysC promoter contained in the chromosome of the parent strain against the gap promoter (gap-RBS) was achieved by conjugation and subsequent screening for homologous recombination events as described in WO03/014330. A strain was obtained which contained in its chromosome the gap promoter including the gap RBS in front of the coding sequence of the lysC gene. Thus the expression of the aspartokinase was placed under the control of the gap promoter.

Strains of Corynebacterium glutamicum carrying the combined gap-RBS-lysC sequence produced significant higher amounts of L-lysine as compared to the parental strains. Enzyme activity analysis of these strains showed a higher aspartate kinase activity.

It was also found that significant overexpression of a number of genes known to be beneficial for L-lysine production (see WO03/014330, Table 1) can be achieved by placing the gap promoter in front of said genes.

In particular expression of enzymes involved in the pentose phosphate pathway (e. g. glucose 6 phosphate dehydrogenase complex encoded by genes zwf and opcA) or involved anaplero anaplerosis (e.g. phosphoenolpyruvate carboxylase encoded by the ppc gene and pyruvate carboxylase encoded by the pyc gene) were increased by cloning the gap promoter in front of said genes. The enhancement of the expression of these genes also resulted in a significant increase of L-lysine production.

## Claims

1. Process for producing L-amino acid, comprising fermenting a microorganism, wherein the expression of a lysC gene encoding a feed back resistant aspartokinase is enhanced by cloning the gap promoter in front of said lysC gene.

## Patentansprüche

1. Verfahren zur L-Aminosäure-Herstellung, umfassend die Fermentation eines Mikroorganismus, wobei die Expression eines eine feedback-resistente Aspartokinase codierenden lysC-Gens durch Klonieren des gap-Promotors vor das lysC-Gen verbessert wird.

## Revendications

1. Procédé de production d'un L-acide aminé, comprenant la fermentation d'un microorganisme, l'expression d'un gène lysC codant pour une aspartokinase résistante à la rétroaction étant augmentée par le clonage du promoteur gap devant ledit gène lysC.
